# EUROPEAN PATENT APPLICATION

(11) **EP 4 201 412 A1**
(43) Date of publication of application: **28.06.2023**
(21) Application number: 21809186.6
(22) Date of filing: 08.04.2021
(51) Int. Cl.: A61K 33/36, A61P 31/14, A23L 33/16

(54) **PREVENTIVE OR THERAPEUTIC PHARMACEUTICAL COMPOSITION FOR CORONAVIRUS INFECTION COMPRISING TETRAARSENIC HEXOXIDE**

(30) Priority: 18.05.2020 KR 20200059410
(71) Applicant: Chemas Co., Ltd., Gyeonggi-do 13815 (KR)
(72) Inventor: BAE, Ill Ju, Jeongseon-gun, Gangwon-do 26112 (KR); KIM, Seong Jin, Seoul 06667 (KR); LYOO, Kwang Soo, Goyang-si, Gyeonggi-do 10360 (KR); YANG, Kyung Min, Incheon 21995 (KR); WU, Zhaoyan, Seoul 08305 (KR); SON, Min Jung, Seoul 06656 (KR); PANG, Kyoungwha, Seoul 06798 (KR)
(74) Representative: Gerauer, Marc Philippé
(86) International application number: PCT/KR2021/004451
(87) International publication number: WO 2021/235695

(57) **Abstract**

Disclosed herein is a pharmaceutical composition comprising tetraarsenic hexoxide as an active ingredient. Having excellent antiviral activity against coronaviruses, the pharmaceutical composition can be advantageously applied to the prevention or treatment of coronavirus diseases.

## Description

### Technical Field

The present disclosure relates to a composition comprising tetraarsenic hexoxide for prevention or treatment of coronavirus disease.

### Background Art

Coronaviruses are a group of related RNA viruses that cause diseases in a wide spectrum of hosts including humans, birds, rodents, and mammals and are larger than any other RNA viruses, with a genome size amounting to up to about 30 kb. The name "coronavirus" refers to the characteristic appearance of virions which have a fringe of surface protein projections creating an image reminiscent of the solar corona or crown.

There are seven strains of coronaviruses that are known to cause diseases in humans. Among them, four strains (HCoV-229E, HCoV-OC43, HCoV-NL63, and HCoV-HKU1), which are named community-acquired respiratory HCoV (CAR HCoV), have a seasonal incidence occurring in the winter and spring months rather than the summer and autumn months in temperature climates over the world and accounts for 10-30 % of the upper respiratory infections in adults. In addition, the coronaviruses SARS-CoV and MERS-CoV which were responsible for the outbreak of severe acute respiratory syndrome (SARS) in 2003 and Middle East respiratory syndrome (MERS) in 2012, respectively, are species transmitted from animals to humans and are known to cause severe respiratory syndromes through lower respiratory infection. From patients suffering from pneumonia, the outbreak of which was first identified in Wuhan, Hubei province, China on December, 2019, a novel coronavirus was detected and given the interim name 2019 novel coronavirus (2019-nCoV), later renamed severe acute respiratory syndrome coronavirus 2 (SARS-CoV-2). The infection syndrome caused by this strain was named coronavirus disease 2019 (COVID-19) by the World Health Organization (WHO) (named "coronavirus disease-19", and abbreviated as "corona-19" in South Korea).

SARS-CoV-2 is a member of the betacoronaviruses that have their origin in bats, like SARS and MERS coronaviruses, and was identified to have the highest genetic similarity (89.1%) to bat SARS-like coronavirus isolates, as analyzed by DNA sequencing. COVID-19 is mostly known for affecting the respiratory tract and is characterized by strong infectivity that begins in the early stage with almost no symptoms appearing. After infected with the virus, patients suffer from sore throat, fever, cough, and breathing difficulties and develop pneumonia. Since the worldwide spread of COVID-19, the World Health Organization (WHO) declared a pandemic on March 11, 2020. As of May 2020, about 4.7 million cases have been confirmed with the death toll amounting to about 0.3 million. The rapid spread of COVID-19 requires urgent development of a therapeutic agent or vaccine for treating or preventing COVID-19.

Leading to the present disclosure, inventive and thorough research conducted by the present inventors to find a therapeutic agent for coronavirus symptoms including COVID-19, resulted in the finding that tetraarsenic hexoxide (As₄O₆) has an excellent therapeutic effect on coronavirus symptoms.

As one of related art documents, Korean Patent Number 272835 discloses that tetraarsenic hexoxide (As₄O₆) exhibits excellent anticancer effects when administered to terminal cancer patients with ovarian cancer, bladder cancer, lung cancer, maxillary sinus cancer, kidney cancer, etc., but with no mention on therapeutic effects on coronavirus diseases.

Chinese Patent Number 101433548 A discloses applicability of arsenic compounds such as arsenic disulfide (As₂S₂), arsenic trisulfide (As₂S₃), tetraarsenic tetrasulfide (As₄S₄), and arsenic trioxide (As₂O₃) to pharmaceutical preparation. It states specific cases in which when applied to patients suffering from viral infections, viral pneumonia, etc., a mixture of arsenic disulfide (As₂S₂), mercuric sulfide (HgS), ox bezoar, antelope horn powder, pearl, goldthread, skullcap, gardenia seeds, a dried Dryobalanops aromatica resin, and Banlangen was observed to show therapeutic effects, but without any mention given on the inhibitory activity of tetraarsenic hexoxide against coronaviruses.

In addition, it is advertised in a prior article (Archives of Toxicology, published online: 09 May 2020) that the homeopathic drug "Arsenicum album 30C" in India can be used for the preventive and prophylactic treatment against COVID-19. However, such a drug is made by sequential dilution of an arsenic trioxide (As₂O₃) mother solution until there is not a trace of As in the solution or even a single water molecule that has encountered any of the original As atoms. It is stated scientists disagree that "Arsenicum album 30C" can act against the new virus, and nowhere is the availability of tetraarsenic hexoxide as a therapeutic agent for coronavirus diseases disclosed in the article.

### [Related Art Documents]

### [Patent Literature]

(Patent literature 1) KR 10-272835
(Patent literature 2) CN 101433548A

### [Non-Patent Literature]

(Non-patent literature 1) Geir Bjørklund, et al. 2020. Arsenic intoxication: general aspects and chelating agents. Archives of Toxicology. Published online: 09 May 2020

### Disclosure of Invention

### Technical Problem

An aspect of the present disclosure is to provide a pharmaceutical composition and a health functional food composition, each comprising tetraarsenic hexoxide (As₄O₆) as an active ingredient for preventing, alleviating, or treating a coronavirus disease.

### Solution to Problem

The present disclosure pertains to a pharmaceutical composition comprising tetraarsenic hexoxide (As₄O₆) as an active ingredient for preventing or treating a coronavirus disease.

The coronavirus disease may be preferably selected from the group consisting of coronavirus disease-19 (COVID-19) caused by infection of severe acute respiratory syndrome coronavirus 2 (SARS-CoV-2), severe acute respiratory syndrome (SARS) caused by infection of severe acute respiratory syndrome coronavirus (SARS-CoV), and Middle East respiratory syndrome (MERS) caused by infection of Middle East respiratory syndrome-related coronavirus (MERS-CoV). Among the above, coronavirus disease-19 (COVID-19) caused by infection of severe acute respiratory syndrome 2 (SARS-CoV-2) may be more preferable.

The present disclosure also exhibits additional aspect of a health functional food composition comprising tetraarsenic hexoxide (As₄O₆) as an active ingredient for preventing or alleviating a coronavirus disease.

Below is the detailed description of the present disclosure.

The present disclosure pertains to a pharmaceutical composition comprising tetraarsenic hexoxide (As₄O₆) as an active ingredient for preventing or treating a coronavirus disease.

As used herein, the term "prevention" refers to any action that suppresses coronavirus infection or delays the onset of a coronavirus disease due to the administration of a composition according to the present disclosure.

As used herein, the term "treatment" refers to any action that attenuates symptoms of coronavirus infection or alters the symptoms in a beneficial sense due to the administration of a composition according to the present disclosure.

The coronavirus disease may be preferably selected from the group consisting of coronavirus disease-19 (COVID-19) caused by infection of severe acute respiratory syndrome coronavirus 2 (SARS-CoV-2), severe acute respiratory syndrome (SARS) caused by infection of severe acute respiratory syndrome coronavirus (SARS-CoV), and Middle East respiratory syndrome (MERS) caused by infection of Middle East respiratory syndrome-related coronavirus (MERS-CoV). Among the above, coronavirus disease-19 (COVID-19) caused by infection of severe acute respiratory syndrome coronavirus 2 (SARS-CoV-2) is more preferably.

Having a remarkable antiviral activity particularly against SARS-CoV-2 causative of coronavirus disease-19, the active ingredient tetraarsenic hexoxide of the present disclosure exhibits an excellent therapeutic effect on coronavirus disease-19. In comparison with other arsenic compounds such as arsenic disulfide (As₂S₂) or arsenic trioxide (As₂O₃), tetraarsenic hexoxide according to the present disclosure has an excellent inhibitory activity against the proliferation of SARS-CoV-2.

Transcriptome analysis of Vero E6 cells infected with SARS-CoV-2 revealed that tetraarsenic hexoxide exhibits antiviral activity against SARS-CoV-2 through the mechanism of upregulating the expression of the genes BAG3, HSPA1B, CRYAB, HMOX1, and HSPH1, all being involved in suppressing viral proliferation and defending viral proliferation-induced cell death. Expression of ZFAND2A that is involved in alleviating arsenic toxicity in cells was also increased. Both leads to the mechanism of downregulated expression of cytokine genes such as CSF1, PDGFA, PDGFB, CXCL1, CXCL2, CXCL3, CXCL8, CXCL10, CCL2, and CCL20, which are involved in inducing inflammatory reaction upon viral infection.

According to usual methods, the pharmaceutical composition of the present disclosure may be formulated into oral dosage forms such as pulvis, granules, tablets, capsules, suspensions, emulsions, syrups, aerosols, etc.; topical agents; suppositories; and sterile injections. Examples of a carrier, an excipient, and a diluent that may be contained in the pharmaceutical composition include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil. When prepared into dosage forms, the pharmaceutical composition is formulated with an ordinary diluent or excipient such as a filler, a thickener, a binder, a humectant, a disintegrant, a surfactant, and so on. Solid preparation for oral administration includes tablet, pill, powder, granules, capsule, and the like. These solid preparations are prepared by mixing tetraarsenic hexoxide of the present disclosure with at least one excipient, such as, starch, calcium carbonate, sucrose or lactose, gelatin, or the like. In addition, lubricants, such as magnesium stearate and talc, can be used in addition to general excipients. Liquid preparation for oral administration includes suspension, liquid for internal use, emulsion, and syrup. Liquid preparation may contain typical diluents, such as water and liquid paraffin, and several excipients, such as humectant, sweetener, aromatic agent, preservative, and the like. Preparation for parenteral administration includes sterile aqueous solution, non-aqueous solvent, suspending agent, emulsifier, lyophilizing agent, and suppository. Examples of the non-aqueous solvent and the suspending agent include propylene glycol, polyethylene glycol, vegetable oils such as olive oil, injectable esters such as ethylolate, and the like. Suppository base materials include Witepsol, Macrogol, Tween 61, cacao butter, laurin butter, glycerogelatin, and the like.

The dose of tetraarsenic hexoxide may vary depending on various factors including the age, sex, and body weight of a subject to be treated, a particular disease or pathological condition to be treated, the severity of a disease or pathological condition, the administration route, and the prescriber's opinion. The determination of dose based on these factors is within the level of a person skilled in the art, and the dose may be generally in the range of approximately 0.01-100 mg/kg/day, more preferably in the range of approximately 0.05-10 mg/kg/day, and far more preferably in the range of approximately 0.05-1 mg/kg/day. The administration may be carried out once a day or several times at divided doses per day. Such doses are not intended to restrict the scope of the present disclosure in any way.

The pharmaceutical composition of the present disclosure may be administered via various routes. All manners of administration can be considered. For example, the administration may be carried out through oral, rectal, intravenous, intramuscular, subcutaneous, endometrial, intracerebroventricular injection.

According to another aspect thereof, the present disclosure also provides a health functional composition for preventing or alleviating a coronavirus disease.

As used herein, the term "health functional food" refers to a food that is prepared and processed by employing a material or ingredient functionally advantageous for the human body. In this context, the "functional" refers to pertaining to an effect that is useful for health through nutritional control or physiological action with respect to the structure and function of the human body.

No particular limitations are imparted to kinds of the health functional food. It may be in the form selected from the group consisting of powder, granule, tablet, capsule, candy, chewing gum, jelly, and beverage. The health functional food of the present disclosure is indented to encompass all the health functional foods used in common sense. The health functional food may further comprise a sitologically acceptable food auxiliary additive. Examples of the sitologically acceptable food auxiliary additive available in the present disclosure include natural carbohydrates such as saccharides, e.g., glucose, fructose, maltose, sucrose, dextrin, cyclodextrin, etc., and sugar alcohols, e.g., xylitol, sorbitol, erythritol, etc.; natural sweeteners such as thaumatin, stevia extracts, etc.; synthetic sweeteners such as saccharin, aspartame, etc.; colorants; pectic acid or a salt thereof; alginic acid or a salt thereof; organic acids; protective colloidal thickeners; pH adjusting agents; stabilizers; preservatives; glycerin; alcohols; and carbonizing agents, but are not limited thereto. The amount of the active ingredient to be mixed may be appropriately determined depending on the purposes thereof and may be 10 parts by weight or less and preferably 5 parts by weight or less on the basis of 100 parts by weight of the health functional food composition. However, the active ingredient may be lower than the lower limit of the range suggested above when ingested for a long period of time for health purposes.

### Advantageous Effects of Invention

Having excellent inhibitory activity against coronaviruses, the composition for preventing or treating a coronavirus disease according to the present disclosure can find advantageous applications in the therapy of coronavirus diseases such as COVID-19, etc.

### Brief Description of Drawings

FIG. 1(A) is an image showing the inhibitory activity of tetraarsenic hexoxide of the present disclosure against SARS-CoV-2 infection-induced cytopathic effect and FIG. 1(B) is an image showing the cytopathic effect resulting from the proliferation of SARS-CoV-2 in the negative control treated with no test substances.
FIG. 2 is a hierarchical clustering heat map for transcriptomes changing after the treatment of tetraarsenic hexoxide in SARS-CoV-2-infected Vero E6 cells.
FIG. 3 shows graphs of gene ontology enrichment results in SARS-CoV-2-infected Vero E6 cells incubated with tetraarsenic hexoxide.
FIG. 4 shows graphs of expression patterns of the genes (BAG3, HSPA1B, CRYAB, HMOX1, 5 ZFAND2A, HSPH1) which are upregulated by treatment with tetraarsenic hexoxide in SARS-CoV-2-infected Vero E6 cells as measured by transcriptome analysis.
FIG. 5 shows graphs of expression patterns of the cytokine genes (CSF1, PDGFA, PDGFB, CXCL1, CXCL2, CXCL3, CXCL8, CXCL10, CCL2, CCL20) which are downregulated by treatment with tetraarsenic hexoxide in SARS-CoV-2-infected Vero E6 cells as measured by transcriptome analysis.
FIG. 6 is an image showing expression levels of the genes (BAG3, ZFAND2A, CRYAB, HSPH1, and HMOX1) which are upregulated by treatment with tetraarsenic hexoxide in SARS-CoV-2-infected Vero E6 cells as analyzed by RT-PCR.
FIG. 7 is an image showing expression levels of the genes (BAG3, ZFAND2A, CRYAB, HSPH1, and HMOX1) which are upregulated in a dose-dependent manner by treatment with tetraarsenic hexoxide in Vero E6 cells as analyzed by RT-PCR.

### Best Mode for Carrying out the Invention

Hereinafter, preferred embodiments of the present disclosure will be described in detail. However, the present disclosure is not limited to the embodiments described herein, but may be embodied in other forms. Rather, the embodiments are provided so that the content introduced herein will be thorough and complete, and will fully convey the spirit of the present disclosure to those skilled in the art.

### <EXAMPLE 1. Assay for Antiviral Activity against Coronaviruses>

Effects of the tetraarsenic hexoxide compound of the present disclosure on coronaviruses were examined through a CPE (cytopathic effect) assay that accounts for comparison of viability between infected cells.

### Example 1-1. Vero cell culture

Vero E6 cells, which are derived from kidney cells extracted from an African green monkey, were seeded at a density of 1×10⁴ cells/well into 96-well plates and incubated overnight in DMEM (Dulbecco's modified Eagle's medium) supplemented with glucose, 10% FBS (fetal bovine serum), 1% PS (penicillin + streptomycin), 1% L-glutamine 200mM, 1% sodium pyruvate 100mM, and nonessential amino acids before being washed with a PBS buffer.

### Example 1-2. Cytotoxicity assay

Cytotoxicity was examined through an MTT assay. The MTT assay is a colorimetric assay for assessing cytotoxicity or viability by taking advantage of the mitochondrial ability to convert the yellow watersoluble substrate MTT tetrazolium into purple insoluble formazan product (3-(4,5-dimethylthiazol-2-yl)-2,5-diphenyl-tetrazolium bromide) by the action of oxidoreductase enzymes present in mitochondria of living cells.

The cultured Vero E6 cells were seeded into 96-well plates. A 1% (w/v) solution of tetraarsenic hexoxide (As₄O₆) in distilled water was 1,000- to 20,000-fold diluted (As₄O₆ 25.27 µM-1.26 µM) in DMEM and each of the dilutions was applied to the 96-well plates in duplicate, followed by incubation for 48 hours. Thereafter, the supernatant was removed and a 5 mg/ml MTT solution was added in an amount of 20 µl to each well. Formazan crystals were formed during incubation at 37°C for 4 hours. After incubation, the supernatant was removed again and DMSO was added in an amount of 100 µl/well to all wells and mixed to completely dissolve the formazan crystals. Incubation at room temperature for 15 minutes made the formazan crystals completely dissolved, followed by reading absorbance at 570 nm (A570 nm) on a microplate.

From the MTT assay results, it was found that the 1% (w/v) tetraarsenic hexoxide (As₄O₆) solution exhibited cytotoxicity leading to cell death until up to 1,000-fold dilution, but the cells survived and did not undergo cell death at 2,000-fold or greater dilutions of the 1% (w/v) tetraarsenic hexoxide (As₄O₆) solution. At least 2,000-fold dilutions of the solution were thus identified to be suitable concentrations for test use.

### Example 1-3. Assay for antiviral efficacy

Vero E6 cells cultured in Example 1-1 were seeded at a density of 2~5×10⁵ cells/well into 8-well plates to which SARS-CoV-2, causative of COVID-19, provided by the Korea Disease Control and Prevention Agency, was then inoculated at a concentration of 10^{1.5}-10^{2.5}TCID₅₀/ml in an amount of 100 µl/well, followed by incubation for 1 hour in a CO₂ incubator to infect the cells with the coronavirus. Afterwards, the culture medium was washed with PBS. 1% (w/v) tetraarsenic hexoxide (As₄O₆) solution was 2,000-, 3,000-, and 4,000-fold diluted (12.64 µM, 8.42 µM, and 6.32 µM) with DMEM and 200 µl of each dilution was added. As a negative control, DMEM free of the test material was added. After 3 days of incubation in an incubator, examination was made of a cytopathic effect (CPE) to determine effective concentrations of the test material. CPE refers to morphological changes in host cells resulting from viral infection. Degenerative morphological changes of cells are known to be associated with viral infection.

The assay for antiviral efficacy identified that the tetraarsenic hexoxide of the present disclosure has an excellent inhibitory effect on the growth of SARS-CoV-2.

**TABLE 1**

| Group | | SARS-CoV-2 inoculated at 10^{2.5}TCID₅₀/ml dose | SARS-CoV-2 inoculated at 10^{1.5}TCID₅₀/ml dose |
|---|---|---|---|
| | | % Inhibition of CPE (cytopathic effect) | % Inhibition of CPE |
| Test Group (tetraarsenic hexoxide treated) | 2,000-fold diluted (12.64µM) | 100% (8/8well) | 1000 (8/8well) |
| | 3,000-fold diluted (8.42µM) | 37.5% (3/8well) | 87.5% (7/8well) |
| | 4,000-fold diluted (6.32µM) | 37.5% (3/8well) | 87.5% (7/8well) |
| Negative Control (tetraarsenic hexoxide not treated) | | 0% (all morphological ly changed) | 0% (all morphological ly changed) |

As can be seen in the table, the proliferation of viruses was 100% inhibited in Vero E6 cell group infected with 10^{2.5}TCID₅₀/ml SARS-CoV-2 when the cells were treated with the 2,000-fold diluted tetraarsenic hexoxide for 3 days.

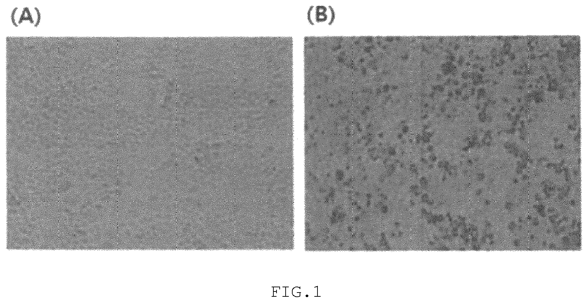

In addition, as the negative control cells, Vero E6 cells infected with 10^{1.5}TCID₅₀/ml of SARS-CoV-2 underwent degenerative structural changes in all wells (8/8 wells) when they were not treated with tetraarsenic hexoxide, but exhibited inhibition against viral growth by 87-100% when treated with tetraarsenic hexoxide. FIG. 1 shows Vero E6 cells infected with 10^{1.5}TCID₅₀/ml of SARS-CoV-2 which exhibited 100% inhibition of CPE in the presence of 12.64 µM tetraarsenic hexoxide (2,000-fold dilution of the 1% (w/v) solution) and all underwent morphological changes-in-the negative control.

Therefore, the pharmaceutical composition comprising tetraarsenic hexoxide as an active ingredient according to the present disclosure has excellent antiviral activity against coronaviruses, especially SARS-CoV-2 and, as such, can find advantageous applications in therapeutic agents for COVID-19, which is caused upon infection of SARS-CoV-2.

### <EXAMPLE 2. Analysis for Transcriptomic Change by Tetraarsenic Hexoxide in Coronavirus-Infected Cells>

An analysis was made for transcripts associated with the antiviral mechanism of tetraarsenic hexoxide upon infection with SARS-CoV-2. To this end, Vero E6 cells derived from kidney cells of African green monkeys were infected with SARS-CoV-2 and then treated with tetraarsenic hexoxide before RNA sequencing analysis. In brief, the same assay procedure as in Example 1-3 was carried out wherein the exception that the cells were infected with 10^{1.5}TCID₅₀/ml SARS-CoV-2 and the tetraarsenic hexoxide solution was 2,000-fold diluted. Used as test groups were a normal control in which Vero E6 cells had been treated with no substances, negative controls in which SARS-CoV-2-infected Vero E6 cells had been treated with DMEM free of test materials for 36 or 72 hours (SARS-Cov-2(36h) and SARS-Cov-2(72h)), and tetraarsenic hexoxide-treated groups in which SARS-CoV-2-infected Vero E6 cells had been treated with tetraarsenic hexoxide for 36 or 72 hours (SARS-Cov-2 + As₄O₆(36h) and SARS-Cov-2 + As₄O₆(72h)). Subsequently, a sample obtained from each test group was subjected to RNA sequencing.

### Example 2-1. RNA isolation and library preparation

From the Vero E6 cell samples prepared as above, RNA was extracted using a TRIzol reagent (Invitrogen, USA) according to the instruction of the manufacturer. The extracted RNA was evaluated for quality and integrity using 2100 Bioanalyzer (Agilent, USA). Only RNA samples with a RIN (RNA integrity number) of 8 or greater were employed. RNA was quantitated using NanoDrop ND-1000 spectrophotometer (NanoDrop Technologies, USA) and libraries of RNA were prepared with the aid of a kit (Illumina TruSeqTM RNA Sample Preparation Kit, Illumina, USA). First, mRNA molecules with poly-A were purified with oligo-dT-attached magnetic beads, and digested RNA fragments were subjected to reverse transcription into first-strand complementary DNA (cDNA) by a reverse transcriptase in the presence of random primers. Then, second-strand cDNA was synthesized using DNA polymerase I and RNaseH (Invitrogen). The cDNA fragments were allowed to go through an end repair process by adding a single A base and then ligating the adapters. The products were then purified and enriched with PCR to create the final cDNA library.

### Example 2-2. RNA sequencing

The cDNA library was subjected to paired-end sequencing on an Illumina instrument (Illumina HiSeq 2000 sequencer, Illumina). From a total of 15 samples, 680,000 reads were created. The paired-end reads were filtered using FastQC and Trimmomatic (ver. 0.32). Subsequently, clean reads were aligned using the Hisat2(ver.2.0.5)-Cufflinks-Cuffmerge-Cuffdiff (ver. 2.2.1) pipeline according to the Tuxedo protocol and quantitated. The reads were mapped to the African green monkey reference genome (ChlSab1.1) downloaded from Ensembl (www.ensembl.org/Chlorocebus_sabaeus/). RNA sequencing detected a total of 27,085 single genes.

### Example 2-3. Heat map clustering analysis

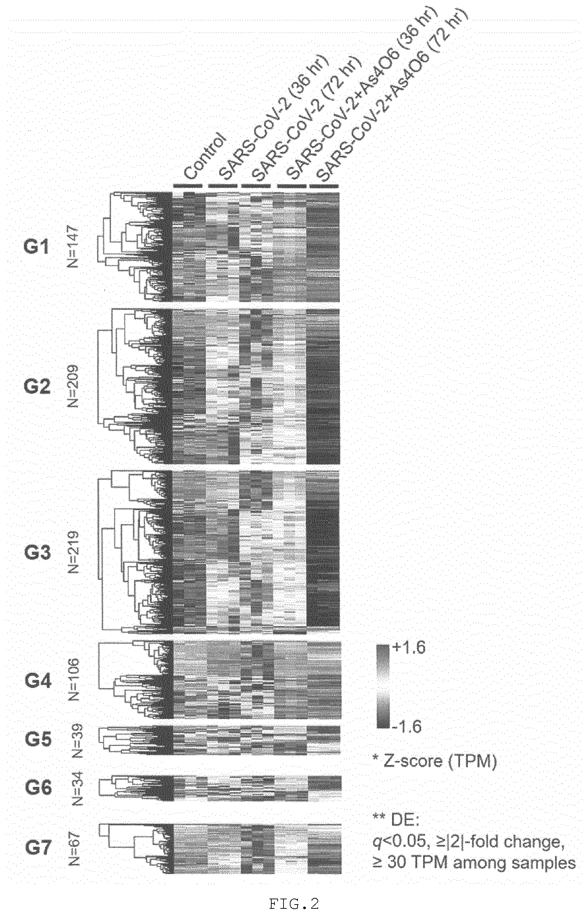

Transcriptomic assay was performed on each test group. Using a heat map, clustered were functionally similar genes that differed in expression level by two fold or greater and statistically showed a q value of 0.05 or lower when transcriptomes from the negative controls in which SARS-CoV-2-infected Vero E6 cells had been incubated (SARS-Cov-2(36h) and SARS-Cov-2(72h)) and the tetraarsenic hexoxide-treated groups in which SARS-CoV-2-infected Vero E6 cells had been treated with tetraarsenic hexoxide (SARS-Cov-2 + As₄O₆(36h) and SARS-Cov-2 + As₄O₆(72h)) were compared to that from the normal control in which Vero E6 cells had been treated with no substances. As a result, a total of 7 groups were divided on the basis of functional similarity of genes: 147 genes for group 1; 209 genes for group 2; 219 genes for group 3; 106 genes for group 4; 39 genes for group 5; 34 genes for group 6; and 67 genes for group 7 (FIG. 2) .

### Example 2-4. Result of gene ontology analysis for responsive genes in SARS-CoV-2-infected Vero E6 cells

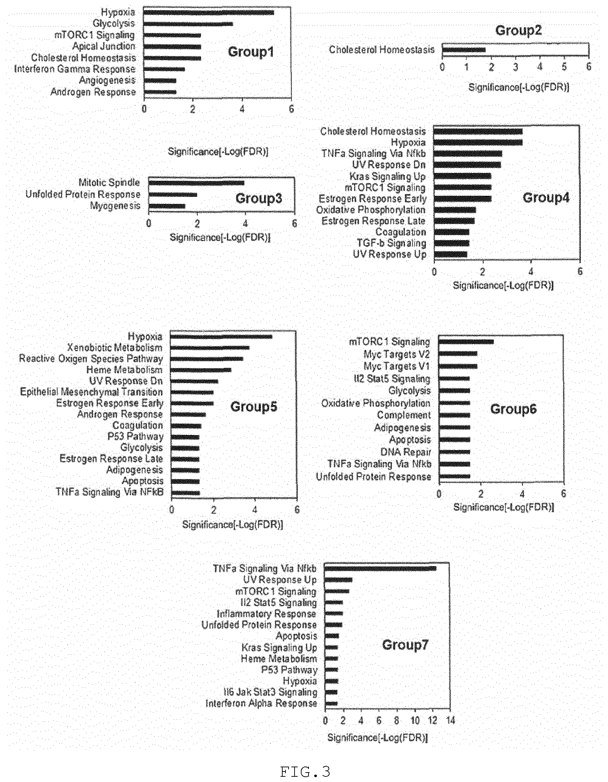

Based on the clustering in Example 2-3, gene ontology (GO) term enrichment was performed for similar genes. Analysis for upper GO terms in each group hierarchically classified in FIG. 2 revealed that GO genes associated with hypoxia, cholesterol homeostasis, TNF-a signaling via NFkB, reactive oxygen species pathway, inflammatory response, IL6-Jak-Stat3 signaling, and interferon alpha response, which are responsible for viral proliferation and inhibition, were regulated by tetraarsenic hexoxide upon infection with SARS-CoV-2 (FIG. 3).

### Example 2-5. Analysis for expression pattern of representative responsive genes in SARS-CoV-2-infected Vero E6 cells

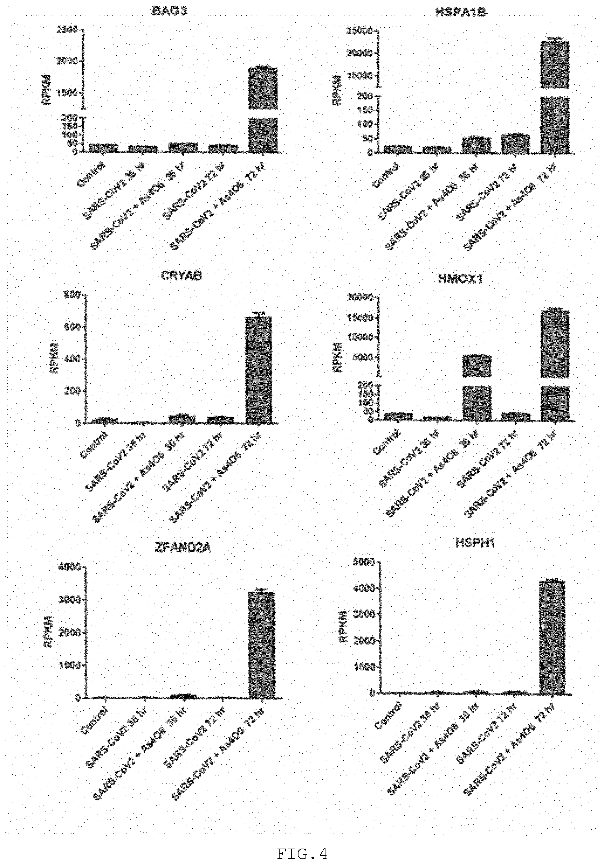

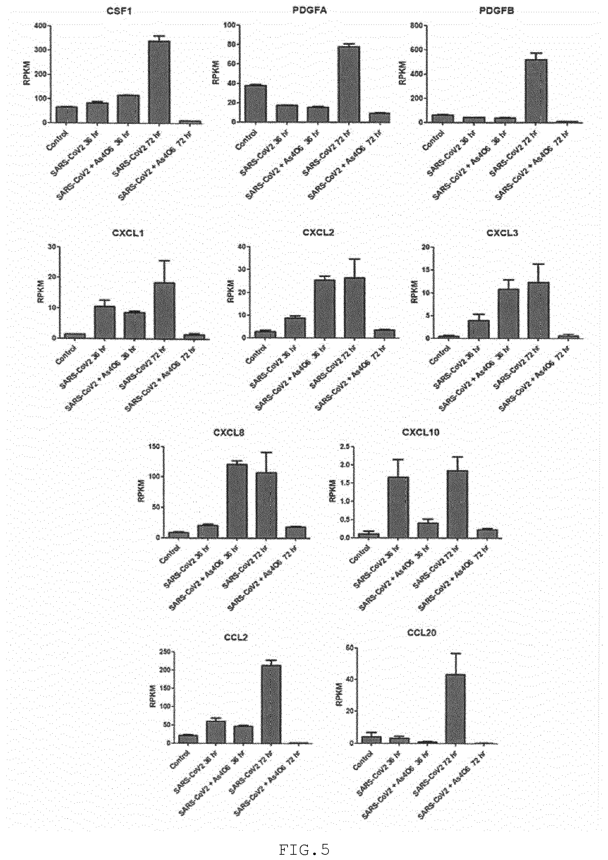

Treatment with tetraarsenic hexoxide upon infection with SARS-CoV-2 was found to regulate genes responsive to viral infection as measured by transcriptomic assay and GO term enrichment. In this context, representative genes responsive to viral infection were analyzed for expression patterns. As a result, treatment with tetraarsenic hexoxide upregulated the expression of the genes BAG3, HSPA1B, CRYAB, HMOX1, and HSPH1, which inhibit viral infection-induced cell death. It also increased the expression of the gene ZFAND2A, which functions to reduced intracellular arsenic toxicity (FIG. 4) and remarkably downregulated the expression of the cytokine genes CSF1, PDGFA, PDGFB, CXCL1, CXCL2, CXCL3, CXCL8, CXCL10, CCL2, and CCL20, which induce inflammatory responses upon viral infection (FIG. 5).

### Example 2-6. Verification using reverse transcription polymerase chain reaction (RT-PCR)

In order to confirm reproducibility of the genes identified through RNA sequencing, RT-PCR was performed on the genes BAG3, CRYAB, HSPH1, HMOX1, and ZFAND2A using the same RNA samples as in the transcriptomic assay.

In detail, RT-PCR was carried out using the primers of Table 2, below, complementary DNA synthesized with reverse transcriptase, and a PCR instrument (TAKARA TP350, TAKARA).

**[TABLE 2]**

| Gene | Primer Sequence (5'→3') | Amplicon Size (bp) |
|---|---|---|
| BAG3 | Forward: ATGACCCATCGAGAATCTGC | 365 |
| | Reverse: GCTTCCACTTTCAGCACTCC | |
| CRYAB | Forward: TTCTTCGGAGAGCACCTGTT | 350 |
| | Reverse: AGGACCCCATCAGATGACAG | |
| HSPH1 | Forward: AGGCCGCTTTGTAGTTCAGA | 345 |
| | Reverse: TTTGCTTTGTCAGCATCTGG | |
| HMOX1 | Forward: GCAGGAAGTCATCCCCTACA | 382 |
| | Reverse: CTGGTCCTTGGTGTCATGTG | |
| ZFAND2A | Forward: GGGGAAGCATTGTTCAGAAA | 381 |
| | Reverse: CTGTGGTCCAAAGGGTGTCT | |
| 18s rRNA | Forward: CCCAACTTCTTAGAGGGACA | 158 |
| | Reverse: TAGTCAAGTTCGACCGTCTT | |

One µg of total RNA was reverse transcribed into cDNA which was then 4-fold diluted in deionized water. Together with 2 µl of the cDNA, 1 µl of tag polymerase, 2 µl of 10X buffer, 2 µl of each primer, and 11 µl of deionized water were mixed to form a total of 20 µl of a reaction mixture. RT-PCR was carried out with 30 cycles of 95°C for 5 min, 58°C for 30 sec, and 72°C for 10 min. Expression levels of target genes were normalized to that of 18s rRNA.

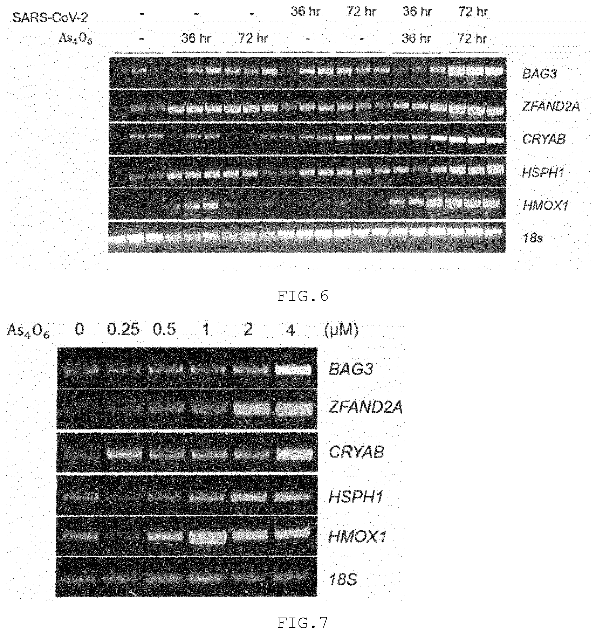

Like the transcriptomic assay, RT-PCR analysis exhibited upregulated expressions of the genes BAG3, CRYAB, HSPH1, and HMOX1, which are associated with cytoprotection responsible for inhibiting virus-induced cell death and viral proliferation, and the gene ZFAND2A, which functions to reduce intracellular arsenic toxicity (FIG. 6). In addition, tetraarsenic hexoxide increased expression levels of the genes mentioned above in a dose-dependent manner in Vero E6 cells that were not infected with SARS-CoV-2 (FIG. 7). Taken together, the data suggest that tetraarsenic hexoxide has antiviral effects on SARS-CoV-2 through the mechanisms of upregulating the expression of genes responsible for inhibiting viral proliferation and defending against viral proliferation-induced cell death, and downregulating the expression of cytokine genes responsible for inducing inflammatory responses to viral infection.

## Claims

1. A pharmaceutical composition, comprising tetraarsenic hexoxide as an active ingredient for preventing or treating a coronavirus disease.

2. The pharmaceutical composition of claim 1, wherein the coronavirus disease is selected from the group consisting of coronavirus disease-19 (COVID-19) caused by infection of severe acute respiratory syndrome coronavirus 2 (SARS-CoV-2), severe acute respiratory syndrome (SARS) caused by infection of severe acute respiratory syndrome coronavirus (SARS-CoV), and Middle East respiratory syndrome (MERS) caused by infection of Middle East respiratory syndrome-related coronavirus (MERS-CoV).

3. The pharmaceutical composition of claim 1, wherein the coronavirus disease is coronavirus disease-19 (COVID-19) caused by infection of severe acute respiratory syndrome 2 (SARS-CoV-2).

4. A health functional food composition, comprising tetraarsenic hexoxide as active ingredient for preventing or alleviating a coronavirus disease.

5. The health functional food composition of claim 4, wherein the coronavirus disease is selected from the group consisting of coronavirus disease-19 (COVID-19) caused by infection of severe acute respiratory syndrome coronavirus 2 (SARS-CoV-2), severe acute respiratory syndrome (SARS) caused by infection of severe acute respiratory syndrome coronavirus (SARS-CoV), and Middle East respiratory syndrome (MERS) caused by infection of Middle East respiratory syndrome-related coronavirus (MERS-CoV).

6. The health functional food composition of claim 4, wherein the coronavirus disease is coronavirus disease-19 (COVID-19) caused by infection of severe acute respiratory syndrome 2 (SARS-CoV-2).
